# EUROPÄISCHE PATENTSCHRIFT

(11) **EP 0 646 106 B1**
(45) Veröffentlichungstag und Bekanntmachung des Hinweises auf die Patenterteilung: **14.10.1998**
(21) Anmeldenummer: 93912919.3
(22) Anmeldetag: 09.06.1993
(51) Int. Cl.: C07C 209/62, C07C 51/06, C07C 55/14, B01D 61/44, C08J 11/16, C08G 69/28, C25B 3/00

(54) **VERFAHREN ZUR GLEICHZEITIGEN HERSTELLUNG VON DICARBONSÄUREN UND DIAMINEN DURCH SPALTUNG VON POLYAMIDEN IN IHRE MONOMEREN BESTANDTEILE**
METHOD FOR THE SIMULTANEOUS PREPARATION OF DICARBOXYLIC ACIDS AND DIAMINES BY DECOMPOSING POLYAMIDES INTO THEIR CONSTITUENT MONOMERS
PROCEDE DE PREPARATION SIMULTANEE D'ACIDES DICARBOXYLIQUES ET DE DIAMINES PAR DECOMPOSITION DE POLYAMIDES EN LEURS CONSTITUANTS MONOMERES

(30) Priorität: 17.06.1992 DE 4219756
(43) Veröffentlichungstag der Anmeldung: 05.04.1995
(73) Patentinhaber: BASF Aktiengesellschaft, 67063 Ludwigshafen (DE)
(72) Erfinder: SEELIGER, Ursula, D-6700 Ludwigshafen (DE); MUELLER, Wolfgang, F., D-6730 Neustadt (DE); HEIMANN, Frank, D-6700 Ludwigshafen (DE); HUBER, Guenther, D-6701 Dannstadt-Schauernheim (DE); HABERMANN, Wolfgang, D-6500 Mainz 1 (DE); VOSS, Hartwig, D-6710 Frankenthal (DE); SIEGEL, Hardo, D-6720 Speyer (DE)
(86) Internationale Anmeldenummer: EP9301448
(87) Internationale Veröffentlichungsnummer: WO9325513

(56) Entgegenhaltungen:
- EP-A- 0 438 369
- WO-A-92/05863
- CH-A- 412 922
- DE-A- 1 088 063
- DE-A- 2 547 101
- DE-A- 3 926 634
- DE-A- 3 926 642
- US-A- 2 840 606
- US-A- 4 781 809
- CHEMICAL ABSTRACTS, vol. 53, no. 2, 25. Januar 1960, Columbus, Ohio, US; abstract no. 14588g, F. CORDIGNOLA 'RECOVERY OF PRIMARY DIAMINES, ESPECIALLY HEXAMETHYLENEDIAMINE FROM POLYAMIDE WASTES' & IT,A,553 182 in der Anmeldung erwähnt

## Beschreibung

Die vorliegende Erfindung betrifft ein Verfahren zur gleichzeitigen Herstellung von Dicarbonsäuren und Diaminen aus
a) Polymerisaten auf der Basis von Polyamiden aus Dicarbonsäuren oder deren Derivaten mit Diaminen, oder
b) Massen, enthaltend im wesentlichen solche Polymerisate.

Die Rückspaltung von Polyamiden wie Polyamid 66 (Polyhexamethylenadipinsäureamid, "PA 66") in ihre monomeren Bestandteile kann in neutralem, saurem oder basischem Milieu durchgeführt werden, wobei im allgemeinen die Spaltung in basischem Milieu u.a. wegen der kürzeren Reaktionszeit vorteilhafter ist.

In der FR-A 926 873 wird die Spaltung von Polyamiden wie PA 66 und PA 610 mit anorganischen Basen, beispielsweise mit einer 10 bis 15 gew.-%igen Alkalimetallhydroxidlösung wie Natronlauge, bei 200°C und einem Druck von etwa 15 bar beschrieben. Danach wird das entstandene Diamin durch Extraktion oder Destillation aus dem Reaktionsgemisch abgetrennt und durch Vakuumdestillation weiter gereinigt. Die freie Dicarbonsäure gewinnt man nach dieser Lehre durch Zugabe einer starken Säure wie Salzsäure zur diaminfreien Reaktionsmischung und anschließendes Ausfällen.

In der IT-A 553 182 wird durch einen Überschuß an 20 gew.-%iger Natronlauge bei 220°C und 25 bar eine Verkürzung der Reaktionszeit gegenüber dem Verfahren aus der FR-A-926 873 erreicht. Das Diamin wird mit n-Butanol aus der wäßrigen Lösung extrahiert. Ein Beispiel betrifft die Abtrennung von zuvor im Polymerisat in Form von Fasern enthaltenem unlöslichem Titandioxid durch Filtration nach der Spaltung. Die Freisetzung der Dicarbonsäure erfolgt ebenfalls durch Zugabe einer starken Mineralsäure.

In der FR-A 1 070 841 wird die Spaltung von PA 66 mit Alkalimetall- oder Erdalkalimetallhydroxidlaugen beschrieben. Die Aufarbeitung der Reaktionsmischung erfolgt nach dieser Schrift zunächst durch Ansäuern mit Schwefelsäure und nachfolgender Abtrennung der ausgefallenen Adipinsäure. Anschließend wird das Filtrat mit Kalilauge versetzt, wobei sich Hexamethylendiamin als ölige Schicht abscheidet und abgetrennt und gereinigt werden kann. Daneben wird auch die Spaltung und Aufarbeitung von Polycaprolactam (PA 6) enthaltenden Poly- und Copolymeren beschrieben.

Die DE-A 1 088 063 beschreibt die Spaltung von PA 66 in einer 10 gew.-%igen methanolischen NaOH-Lösung. Das erhaltene Dinatriumadipat wird nach dieser Schrift durch Ansäuern in die freie Säure überführt, Hexamethylendiamin ("HMD") kann durch Destillation in reiner Form gewonnen werden.

Die Spaltung von PA 66 in Dinatriumadipat und HMD in einem zweiphasigen C₃-C₈-Alkanol-Wasser-Gemisch beschreibt die US-A 2 840 606. Das HMD wird nach dieser Lehre durch Abtrennen aus der Alkoholphase durch Destillation isoliert. Adipinsäure gewinnt man durch Ansäuern der wäßrigen Phase mit Schwefelsäure. Eine Reinigung der Adipinsäure kann durch Kristallisation erfolgen.

Die DE-A 39 26 642 beschreibt ein Verfahren und eine Vorrichtung, basierend auf einer Vierkammerelektrolysezelle, zur Gewinnung einer Säure aus ihrem Salz. Reaktionsparameter und Beispiele werden in der DE-A 39 26 642 nicht genannt.

Allen diesen Verfahren gemeinsam ist die Gewinnung von Adipinsäure durch Ansäuern der entsprechenden Alkalimetall- oder Erdalkalimetallsalzlösungen. Das hierbei zwangsläufig mitgebildete anorganische Salz, meist Natriumchlorid oder Natriumsulfat, stört einerseits bei der Reinigung der Dicarbonsäure durch Kristallisation, da es diese behindert, und andererseits führen diese Salze zu großen Problemen bei ihrer Entsorgung.

Des weiteren ist von Nachteil, daß mit den beschriebenen Verfahren technisch verwendete, beispielsweise faserverstärkte, mineralgefüllte und/oder schlagzähmodifizierte PA 66 enthaltende Formmassen nicht in geeigneter Weise aufgearbeitet werden können, weil die vielfältigen Zusatzstoffe die Durchführung der beschriebenen Verfahren behindern würden.

Der Erfindung lag daher die Aufgabe zugrunde, ein Verfahren zur gleichzeitigen Herstellung von Dicarbonsäuren und Diaminen zur Verfügung zu stellen, bei dem die oben genannten Nachteile nicht auftreten.

Demgemäß wurde ein Verfahren zur gleichzeitigen Herstellung von Dicarbonsäuren und Diaminen aus
a) Polymerisaten auf der Basis von Polyamiden aus Dicarbonsäuren oder deren Derivate mit Diaminen, oder
b) Massen, enthaltend im wesentlichen solche Polymerisate, gefunden,
das sich dadurch auszeichnet, daß man 1) diese Polymerisate in ihre monomeren Bestandteile spaltet, wobei man diese Polymerisate mit einer Base in einer Alkohol-Wasser Mischung, die von 5 bis 40 Gew.-% Wasser enthält, behandelt und wobei man eine flüssige und eine feste Phase erhält und 2) die feste von der flüssigen Phase trennt und die flüssige Phase, die das Diamin enthält, erhält und 3) die bei der Spaltung erhaltenen Dicarbonsäuresalze der Adipinsäure oder Sebacinsäure elektrochemisch in die entsprechenden Dicarbonsäuren und Basen überführt.

Als Polymerisate auf der Basis von Polyamiden aus Dicarbonsäuren oder deren Derivaten, beispielsweise den entsprechenden Säurehalogeniden, bevorzugt den Säurechloriden, mit Diaminen eignen sich nach den bisherigen Beobachtungen Polyhexamethylenadipinsäureamid, Polyhexamethylensebacinsäureamid und Polytetramethylenadipinsäureamid, bevorzugt Polyhexamethylenadipinsäureamid.

Als Massen, enthaltend im wesentlichen, d.h. zu mindestens 50 Gew.-%, solche Polymerisate, kommen beispielsweise auch Copolyamide mit PA 66 sowie PA 66 oder Copolyamide mit PA 66, enthaltend Fasern und/oder Zusatzstoffe, in Betracht.

Als Basen zur Spaltung der Polymerisate verwendet man in der Regel Alkalimetallhydroxide wie Lithiumhydroxid, Natriumhydroxid und Kaliumhydroxid, bevorzugt Natriumhydroxid, oder deren Mischungen, vorzugsweise eine Mischung aus Natrium- und Kaliumhydroxid.

Zweckmäßigerweise setzt man 1,8 bis 4,0, bevorzugt 2,0 bis 3,0, Äquivalente Alkalimetallhydroxid pro wiederkehrende Einheit im Polymeren, beispielsweise -[-(CH₂)₄-CO-NH-(CH₂)₆-NH-CO-]- bei PA 66, ein. Verwendet man weniger als 1,8 Äquivalente der Base, so erhält man in der Regel unerwünschte Anteile an Oligomeren. Bei mehr als 4,0 Äquivalenten der Base pro wiederkehrende Einheit erhält man im allgemeinen, besonders bei glasfaserverstärkten und/oder mineralgefüllten Polyamid-Formmassen, in zu hohem Maße Abbaureaktionen der Glasfasern oder der mineralischen Füllstoffe.

Erfindungsgemäß setzt man die Base in einer Alkanol-Wasser-Mischung ein, die von 5 bis 40, besonders bevorzugt von 10 bis 30 Gew.-%, Wasser enthält.

Als C₁-C₄-Alkanole kann man im allgemeinen Methanol, Ethanol, n-, i- Propanol, n-Butanol, bevorzugt Methanol, Ethanol und i-Propanol,verwenden.

Die Umsetzung nimmt man in der Regel bei einer Temperatur im Bereich von 100 bis 300, bevorzugt von 140 bis 220°C, vor. Der Druck liegt hierbei im allgemeinen im Bereich von 0,08 bis 15 MPa, wobei man auch außerhalb dieses Druckbereiches arbeiten kann. Bevorzugt arbeitet man unter dem sich bei den Reaktionsbedingungen einstellenden Druck.

Der pH-Wert der Reaktionsmischung ist im allgemeinen, bedingt durch das Alkalimetallhydroxid, größer als 7.

Die Dauer der Reaktion hängt im wesentlichen von den Konzentrationen der Ausgangsstoffe, der Temperatur und dem Druck ab und liegt in der Regel im Bereich von 0,5 bis 15, vorzugsweise von 1 bis 10 h.

Die Spaltung der erfindungsgemäß eingesetzten Polymerisate mit einer Base kann man kontinuierlich oder diskontinuierlich durchführen.

Die Spaltung der Polymerisate durch eine Base kann man in den üblichen Vorrichtungen mit oder ohne Rührer vornehmen, wobei man bevorzugt Druckbehälter mit einem Rührersystem, das besonders gut für Feststoffdispergierung geeignet ist, beispielsweise ein Propellerrührer oder ein Kreuzbalkenrührer, verwendet.

In einer bevorzugten Ausführungsform zerkleinert man das eingesetzte Polymerisat bzw. polyamidhaltige Massen mechanisch auf eine mittlere Teilchengröße von 0,1 bis 50, bevorzugt von 1 bis 10 mm, bevor man es bzw. sie der Spaltung zuführt. Die Zerkleinerung kann man in einer handelsüblichen Mühle, beispielsweise in einer Schneidmühle, oder, bevorzugt, insbesondere wenn die eingesetzten Massen harte Materialien wie Metalleinlegeteile, z.B. Schrauben, enthalten, in einer Hammermühle.

Aus dem auf diese Weise zerkleinerten Material kann man vorhandene Metallteile in einem Trockentrennverfahren mit einem sogenannten Luftherd, bevorzugt mit anschließender Induktionsabscheidung, z.B. mit Hilfe eines Freifall-Rohrabscheiders, zur vollständigen Abtrennung der Metallteile, oder in einem Naßtrennverfahren, beispielsweise mittels eines Hydrozyklons, entfernen.

In einer besonders bevorzugten Ausführungsform zerkleinert man die einzusetzenden Polymerisate bzw. Massen auf eine Größe von maximal 50 mm Länge in einer Hammermühle, trennt gewünschtenfalls vorhandene Metallteile ab, und zerkleinert anschließend das von Metallteilen befreite Mahlgut auf eine Größe im Bereich von 5 bis 12 mm auf einer Schneidmühle. Gegebenenfalls kann man daran anschließend das so aufbereitete Polymerisat bzw. polymerisathaltige Masse noch waschen und trocknen, bevor man es bzw. sie der Spaltung durch eine Base zuführt.

Die nach der Spaltung der Polyamide erhaltene Reaktionsmischung besteht aus einer flüssigen Phase, enthaltend das Diamin, und einer festen Phase, enthaltend das ausgefallene Dicarbonsäuresalz sowie unlösliche Bestandteile.

Erfindungsgemäß trennt man sodann die feste Phase von der flüssigen Phase ab.

Als Verfahren zum Abtrennen der festen Phase eignen sich bekannte Verfahren wie Filtration, Sedimentation oder Zentrifugieren.

Als unlösliche Bestandteile seien beispielhaft genannt Glasfasern, Kohlenstoffasern, Ruß, Mineralien und Kautschuk sowie zuvor nicht oder nicht vollständig abgetrennte Metalle, soweit sie nicht durch die Base in Lösung gebracht wurden.

Die abgetrennte feste Phase kann man gewünschtenfalls in einem weiteren Verfahrensschritt mit zweckmäßigerweise einem organischen Lösungsmittel waschen, bevorzugt mit einem C₁-C₄-Alkohol wie Methanol, Ethanol, n-, i-Propanol, sowie Mischungen davon, oder einer Mischung eines C₁-C₄-Alkohols mit einem Gehalt von 0 bis 30 Gew.-% an Wasser, vorzugsweise mit der bei der Spaltung verwendeten Lösungsmittelkomponente, besonders bevorzugt in einem reinen C₁-C₄-Alkohol.

Das Waschen kann man beispielsweise mit der für die Abtrennung eingesetzten Vorrichtung wie Bandfilter, Zentrifuge, Filterpresse, und Zentrifugal-Scheibendruckfilter, vorzugsweise Zentrifuge und Filterpresse, und/oder anderen, für diesen Zweck geeigneten Vorrichtungen wie einem Dekanter, durchführen. Zweckmäßig führt man den Waschvorgang in mehreren Stufen durch, wobei man nach jeder Stufe die nach dem Waschvorgang abgetrennten unlöslichen Bestandteile vorzugsweise jeweils mit dem verwendeten Waschmedium innig mischt, um den Verlust an löslichen Bestandteilen so gering wie möglich zu halten. Besonders bevorzugt setzt man eine Vorrichtung ein, in der die unlöslichen Bestandteile im Gegenstrom gewaschen werden können.

In einer bevorzugten Ausführungsform kann man die flüssige Phase der nach der Spaltung erhaltenen Reaktionsmischung gewünschtenfalls zusammen mit den flüssigen Phasen aus den Waschoperationen der festen Phase vereinigen und als Lösemittel in der Hydrolyse einsetzen, wobei man vorher das Diamin gewünschtenfalls ganz oder teilweise entfernen kann. Besonders bevorzugt führt man die flüssigen Phasen aus den Waschoperationen der festen Phase direkt in die Hydrolysestufe zurück.

Eine weitere bevorzugte Ausführungsform besteht darin, daß man vorzugsweise nach der Abtrennung der festen Phase die bei der Spaltung in die monomeren Bestandteile erhaltenen Diamine abtrennt.

Die Abtrennung der Diamine aus der flüssigen Phase der aus der Spaltung erhaltenen Reaktionsmischung kann man nach bekannten Verfahren wie Destillation, bevorzugt Rektifikation, oder Extraktion durchführen.

In der Regel werden dabei vor der Abtrennung der Diamine leichtflüchtige Komponenten, in erster Linie die als Lösungsmittel eingesetzten Alkohol-Wasser-Mischungen, vorzugsweise durch Destillation, abgetrennt. Diese Destillation kann man in an sich bekannten Verdampfungsapparaturen, wie Dünnschicht- oder Fallfilmverdampfern, ein- oder mehrstufig, vorzugsweise mehrstufig, oder in einer Rektifikationskolonne durchführen.

Das durch diese Abtrennung erhaltene Lösungsmittel kann man direkt bei der Spaltung der Polymerisate wiedereinsetzen. Man kann es aber auch zuvor, zweckmäßig durch eine Rektifikation, von bei der Spaltung störenden Verunreinigungen befreien. Des weiteren kann es wünschenswert sein, Wasser auf diesem Wege abzutrennen, um auf diesem Wege eine geeignete Zusammensetzung der Alkohol-Wasser-Mischung zu erreichen. Die Rektifikation führt man im allgemeinen in an sich bekannten Apparaturen wie Bodenkolonnen oder Kolonnen mit geordneten oder ungeordneten Packungen durch.

Die Isolation der Diamine führt man in der Regel in an sich bekannten Apparaturen durch, vorzugsweise Bodenkolonnen oder mit Packungen gefüllten Kolonnen, wenn man rektifiziert.

In einer bevorzugten Ausführung erhält man die Diamine als dampfförmigen Seitenabzug im Abtriebsteil der Rektifikationskolonne, oder, ebenfalls bevorzugt, als flüssigen Seitenabzug im Verstärkungsteil der Rektifikationskolonne. Die Rektifikation erfolgt im allgemeinen bei einem Druck im Bereich von 10 bis 100 kPa, bevorzugt von 50 bis 80 kPa.

Aus dem Rektifikationsrückstand kann man gewünschtenfalls den eventuell noch vorhandenen Rest an Diamin in einem weiteren Destillationsschritt, beispielsweise mit einem Dünnschichtverdampfer, bei einem Druck im Bereich von 0,5 bis 50, bevorzugt von 2 bis 30 kPa, bevorzugt in einem zwangsgereinigten Schaufeleindampfer, da hierbei die nicht verdampfbaren Rückstände als Feststoff ausgetragen werden können, abtrennen.

Aus der nach der Spaltung der Polymerisate abgetrennten, gegebenenfalls gewaschenen festen Phase löst man im allgemeinen, beispielsweise aus dem Filterkuchen, das Dicarbonsäuresalz durch Versetzen mit Wasser heraus. Hierzu kann es gegebenenfalls erforderlich sein, Wasser mit einer Temperatur, die höher als die Raumtemperatur liegt, einzusetzen, z.B. auch Wasserdampf.

Diesen Verfahrensschritt kann man je nach Wahl des Verfahrens zur Abtrennung unlöslicher Bestandteile in der auch zur Filtration bzw. zum Waschen der unlöslichen Bestandteile verwendeten Vorrichtung, oder mit weiteren, hierfür geeigneten Vorrichtungen wie Bandfilter, Zentrifuge, Filterpresse und Zentrifugal-Scheibendruckfilter, vorgenommen werden. Gewünschtenfalls mischt man die Wasserphase mit den unlöslichen Bestandteilen innig, z.B. mit einem Intensivmischer, bevor man sie voneinander trennt. Gewünschtenfalls wiederholt man diesen Verfahrensschritt zwei- bis dreimal, um eine möglichst hohe Ausbeute an Dicarbonsäuresalz zu erhalten.

In einer besonders bevorzugten Ausführungsform kann man eine möglichst konzentrierte wäßrige Lösung des Dicarbonsäuresalzes erhalten, wenn man das Dicarbonsäuresalz aus der festen Phase in 2 bis 8, bevorzugt in 3 bis 4, Stufen mit einer Dicarbonsäuresalzlösung herauslöst. Auf diese Weise konzentriert man im allgemeinen die eingesetzte Dicarbonsäuresalzlösung auf und spart energie- und kostenaufwendige Konzentrierungsverfahren wie Eindampfen ein. Zweckmäßig setzt man in der ersten Stufe eine konzentriertere Dicarbonsäuresalzlösung ein als in der zweiten, in der zweiten Stufe eine konzentriertere Dicarbonsäuresalzlösung als in der dritten, usw. In der letzten Stufe setzt man in der Regel vollentsalztes Wasser ein. Bevorzugt verwendet man die am höchsten konzentrierte Lösung zur weiteren Verarbeitung (elektrochemische Behandlung), während man die anderen, weniger konzentrierten Lösungen bis zum nächsten Waschvorgang aufbewahrt bzw. bei kontinuierlicher Verfahrensweise des Waschvorganges, gleich weiterverwendet. Im allgemeinen enthält die Waschlösung der ersten Stufe das Dicarbonsäuresalz in einer Konzentration im Bereich von 5 bis 40, bevorzugt von 15 bis 30 Gew.-%.

Das wasserlösliche Extrakt, bzw. die vereinigten wasserlöslichen Extrakte, kann man anschließend einer Destillation, gegebenenfalls unter vermindertem Druck, unterwerfen, um möglicherweise darin noch vorhandene Reste an Alkoholen und/oder anderen flüchtigen organischen Stoffen zu entfernen. Des weiteren kann es vorteilhaft sein, die wäßrige Lösung vor der elektrochemischen Behandlung durch Entfernen des Wassers mittels Destillation aufzukonzentrieren.

Die nach dem Lösen des Dicarbonsäuresalzes gegebenenfalls erhaltenen unlöslichen Bestandteile können gewünschtenfalls nach Trocknung als Füllstoffe weiterverwendet werden.

Die elektrochemische Behandlung störenden Verunreinigungen wie Erdalkalimetallkationen, Kieselsäure-, Polyphosphatanionen oder hochmolekulare organische Aminverbindungen, kann man zweckmäßig aus den von unlöslichen Bestandteilen und Diaminen befreiten wäßrigen Lösungen, enthaltend im wesentlichen die Dicarbonsäuresalze, entfernen, indem man diese Lösungen mit Adsorptionsmitteln und/oder geeigneten Fällungsmitteln behandelt.

Als Adsorptionsmittel setzt man bevorzugt Aktivkohle, Anthrazit, calcinierte Kokse und makroporöse organische Ionenaustauscher sowie weitere anorganische Adsorbentien ein. Als Fällungsmittel kann man Carbonate der Alkalimetalle und/oder Ammoniumcarbonate einsetzen.

Nach den bisherigen Beobachtungen hat die Art der elektrochemischen Behandlung keinen prinzipiellen Einfluß auf das erfindungsgemäße Verfahren.

Beispielhaft genannt seien die folgenden Verfahrensvarianten (a) bis (f):
(a) Bei dieser Verfahrensweise kann man die Spaltung des Dicarbonsäuresalzes in die entsprechende Dicarbonsäure und die entsprechende Base in einer zweigeteilten Elektrodialysezelle mit bipolaren Membranen durchführen. Im allgemeinen ist die Elektrodialysezelle so aufgebaut, daß zwischen Anode und Kathode 1 bis 200, bevorzugt 20 bis 70 Elektrodialyseeinheiten zusammengefaßt werden, die voneinander durch bipolare Membranen getrennt sind. Die bipolaren Membranen trennt man voneinander durch Kationenaustauschermembranen ab, wodurch der folgende Aufbau in einer Elektrodialyseeinheit vorliegt: bipolare Membran (anodenseitig) - Anolytkammer - Kationenaustauschermembran - Katolytkammer - bipolare Membran (Kathodenseitig). Die einzelnen Elektrodialyseeinheiten schaltet man zweckmäßig elektrisch in Reihe.
   Bei dieser Verfahrensweise führt man zweckmäßigerweise die wäßrige Dicarbonsäuresalzlösung der Anolytkammer zu. Im elektrischen Feld einer angelegten Gleichspannung wandern im allgemeinen die Alkalimetallkationen durch die Kationenaustauschermembran in die Katolytkammer. Die zur Kompensation der getrennten Ladungen erforderlichen Hydroxyl-Anionen, die durch Dissoziation des Wassers in den bipolaren Membranen gebildet werden, stammen aus der Kathodenseitigen bipolaren Membran. Auf diese Weise reichert sich in der Katolytkammer die entsprechende Alkalimetallhydroxid-Lösung an. In der Anolytkammer kann das Dicarbonsäureanion mit den Wasserstoffionen, die aus der anodenseitigen bipolaren Membran stammen, die freie Dicarbonsäure bilden.
   Vorteilhaft führt man die Dicarbonsäuresalzlösung parallel den Anolytkammern zu. Die Produktströme aus den Anolytkammern, enthaltend die freie Säure und nicht umgesetztes Dicarbonsäuresalz, sowie die Produktströme der Katolytkammern faßt man zweckmäßig jeweils zusammen. Die freie Dicarbonsäure gewinnt man in der Regel durch Auskristallisieren aus den zusammengeführten Produktströmen der Anolytkammer, ohne daß das entsprechende Dicarbonsäuresalz, das man vorzugsweise erneut der Elektrodialyse unterwirft, mitausfällt.
   Die Elektrodialyse kann man sowohl kontinuierlich als auch diskontinuierlich durchführen. Eine bevorzugte Ausführungsform bei der kontinuierlichen Verfahrensweise mit mehreren Elektrodialysezellen besteht darin, daß man den Gesamtumsatz auf 2 bis 20, bevorzugt 4 bis 6 Elektrodialysezellen verteilt, und pro Elektrodialysezelle nur einen Teilumsatz vornimmt.
   Besonders vorteilhaft führt man dabei die Stoffströme nach dem Gegenstromprinzip. Der Ablauf der Anolytkammer bildet den Zulauf der darauffolgenden Anolytkammer usw., so daß der Ablauf der letzten Anolytkammer mit Dicarbonsäure angereichert und mit Dicarbonsäuresalz abgereichert ist. Der Ablauf der letzten Katolytkammer, enthaltend eine niedrige Konzentration an Alkalimetallhydroxid, bildet den Zulauf der vorletzten Katolytkammer usw., so daß in der ersten Einheit eine hohe Konzentration an Dicarbonsäuresalz in der Anolytkammer und eine hohe Alkalimetallhydroxidkonzentration in der Katolytkammer vorliegen. Damit erreicht man, daß die Konzentrationsunterschiede an Alkalimetallhydroxid in den Anolyt- und Katolytkammern pro Einheit klein sind. Letztlich führt dies in der Regel zu einer Energieeinsparung durch eine höhere Stromausbeute und im Mittel zu niedrigeren Zellspannungen.
   Die Stromdichten liegen im allgemeinen im Bereich von 0,1 bis 2, bevorzugt von 0,5 bis 1,0 kA/m². Die Zellspannung beträgt in der Regel pro Elektrodialyseeinheit 3 bis 8 V.
   Die pH-Werte liegen im allgemeinen in den Anolytkammern im Bereich von 2 bis 10, in den Katolytkammern im Bereich größer als 13.
   Die Kammerbreite beträgt in der Regel 0,2 bis 5, bevorzugt 0,5 bis 1 mm.
   Die Temperatur während der Elektrodialyse wählt man im allgemeinen im Bereich von 40 bis 110, vorzugsweise von 65 bis 90°C.
   Die Strömungsgeschwindigkeiten der Zu- und Abläufe liegt im allgemeinen im Bereich von 0,05 bis 0,2 m/sec.
   Die Konzentration an eingesetztem Dicarbonsäuresalz beträgt in der Regel 5 bis 40 Gew.-%, bevorzugt 10 bis 20 Gew.-%.
   Gewünschtenfalls kann man die Leitfähigkeit im Anolytsystem durch Zusätze von Salzen oder Säuren wie Natriumsulfat oder Schwefelsäure erhöhen. Solche Stoffe setzt man im allgemeinen im Bereich von 0,1 bis 10 Gew.-%, bevorzugt von 1 bis 6 Gew.-%, bezogen auf das Gesamtgewicht der in der Anolytkammer befindlichen Lösung, ein.
   Der Katolytkammer kann man zweckmäßig die Stoffe, die im Betrieb erhalten werden, vorzugsweise das entsprechende Alkalimetallhydroxid wie Natrium- und Kaliumhydroxid, vorzugsweise Natriumhydroxid, zusetzen.
   Als Zulauf in die Katolytkammer verwendet man in der Regel vollentsalztes Wasser ("VE-Wasser"), wobei man vorzugsweise zu Beginn 1 bis 25, bevorzugt 5 bis 10 gew.-%ige, bei der Elektrodialyse entstehende Alkalimetallhydroxidlösung einsetzt.
(b) Eine dreigeteilte Elektrodialysezelle mit bipolaren Membranen hat den Vorteil gegenüber der unter (a) aufgeführten Verfahrensweise, daß keine hohen Anforderungen an die Reinheit der Einsatzstoffe gestellt zu werden brauchen. Des weiteren erhält man im allgemeinen wesentlich geringere Salzanteile sowohl in der erhaltenen Dicarbonsäurelösung als auch in der entsprechenden Alkalimetallhydroxidlösung.
   Das Dreikammersystem enthält neben einer Kationenaustauschermembran eine Anionenaustauschermembran, so daß sich für eine Elektrodialyseeinheit folgender Aufbau ergibt: bipolare Membran (anodenseitig) - Anolytkammer - Anionenaustauschermembran - Mittelkammer - Kationenaustauschermembran - Katolytkammer - bipolare Membran (kathodenseitig).
   Die Dicarbonsäuresalzlösung führt man zweckmäßig in die Mittelkammer zu. In einem durch Gleichspannung angelegten elektrischen Feld wandern in der Regel die Dicarbonsäureanionen durch die Anionenaustauschermembran in die Anolytkammer, wo sie mit den dort vorhandenen Wasserstoffionen die freie Säure bilden können. Abgesehen von Selektivitätsverlusten der Anionenaustauschermembran kann man der Anolytkammer die freie Säure salzfrei entnehmen. In der Katolytkammer fällt wie bei (a) die Alkalimetallhydroxidlösung an. Den Ablauf der Mittelkammer, enthaltend noch Restmengen an Dicarbonsäuresalz kann man entsorgen oder zweckmäßig dem Zulaufstrom zur Dicarbonsäuresalzauflösung (In-Lösung-bringen des bei der Spaltung ausgefallenen Dicarbonsäuresalzes) zuführen. Analog zu (a) kann man die Stoffströme im Gegenstromprinzip führen, um die Stromausbeute zu erhöhen.
   Zur Erhöhung der Leitfähigkeit kann man der Anolytkammer beispielsweise eine Oxosäure wie Schwefelsäure, Phosphorsäure und Salpetersäure zusetzen.
   Der Katolytkammer kann man zweckmäßig die Stoffe, die im Betrieb erhalten werden, vorzugsweise das entsprechende Alkalimetallhydroxid wie Natrium- und Kaliumhydroxid, vorzugsweise Natriumhydroxid, zusetzen. Im übrigen kann man das Verfahren nach (b) unter den gleichen Bedingungen wie unter (a) beschrieben durchführen.
(c) Prinzipiell kann man auch Elektrodialysezellen mit vier Kammern verwenden. Der Aufbau gleicht im allgemeinen dem einer Elektrodialysezelle mit drei Kammern, mit dem Unterschied, daß zum Schutz der bipolaren Membranen vor möglichen störenden Verunreinigungen eine weitere Ionenaustauschermembran, vorzugsweise eine Kationenaustauschermembran, eingebaut ist. Es liegt in der Regel folgender Aufbau in einer Elektrodialyseeinheit vor: bipolare Membran (anodenseitig) - Anolytkammer - Kationenaustauschermembran - anodennahe Mittelkammer - Anionenaustauschermembran - kathodennahe Mittelkammer - Kationenaustauschermembran - Katolytkammer - bipolare Membran (kathodenseitig).
   Zweckmäßig führt man die Dicarbonsäuresalzlösung der kathodennahen Mittelkammer zu, und führt aus der anodennahen Mittelkammer die Dicarbonsäurelösung und aus der Kathodenkammer die Alkalimetallhydroxidlösung ab.
   Im übrigen kann man das Verfahren nach (c) unter den gleichen Bedingungen wie unter (b) beschrieben durchführen.
(d) Die elektrochemische Spaltung der Dicarbonsäuresalze in Dicarbonsäure und die betreffende Base kann man nach einer weiteren Ausführungsform in einer an sich aus der Chlor--Alkalielektrolyse bekannten zweigeteilten Membranelektrolysezelle durchführen. Die Membranelektrolysezelle ist im allgemeinen so aufgebaut, daß 1 bis 100, bevorzugt 20 bis 70 Elektrolyseeinheiten zu einem Zellblock zusammengefaßt werden. Dabei können die einzelnen Elektrolyseeinheiten elektrisch in Reihe geschaltet werden, indem man die Kathode einer Einheit mit der Anode der nächsten Einheit elektrisch leitend verbindet, oder intern verbundene bipolare Elektroden einsetzt. Die Produktzufuhr und die Produktabfuhr erfolgt in der Regel über Sammelleitungen für jeden Kammertyp getrennt. Die zweigeteilte Membranelektrolyseeinheit ist im allgemeinen in Richtung von Anode zur Kathode wie folgt aufgebaut:
   - Anode-Anolytkammer-Kationenaustauschermembran-Katolytkammer-Kathode.
   Die wäßrige Dicarbonsäuresalzlösung wird zweckmäßig der Anolytkammer zugeführt. Im elektrischen Feld der angelegten Gleichspannung werden im allgemeinen die Alkalimetallkationen durch die Kationenaustauschermembran in die Katolytkammer überführt und dort zu Lauge umgesetzt. Die zur Kompensation der getrennten Ladungen erforderlichen Hydroxylanionen werden bei der Kathodenreaktion freigesetzt. Die Kathodenreaktion kann zum Beispiel die katodische Wasserstoffabscheidung oder zum Beispiel eine katodische Reduktion von Sauerstoff sein. In der Anolytkammer verbleibt im allgemeinen der organische Säurerest, der mit dem Wasserstoffionen bzw. deren hydratisierte Formen, die bei der Anodenreaktion frei werden, die entsprechende freie Säure bildet. Als Anodenreaktion sei zum Beispiel die anodische Sauerstoffabscheidung oder die anodische Oxidation von Wasserstoff genannt. In der Anodenkammer reichert sich somit in der Regel das Salz ab und der Gehalt an freier Dicarbonsäure nimmt zu.
   Die Membranelektrolyse kann sowohl diskontinuierlich, als auch kontinuierlich durchgeführt werden. Bei der kontinuierlichen Fahrweise bietet es sich an den Umsatz auf 2 bis 20, bevorzugt 4 bis 6, zu verteilen und die Stoffströme nach dem Gegenstromprinzip zu führen (s. (a)).
   Die eingesetzte Dicarbonsäuresalzlösung, gegebenenfalls enthaltend mehrere solcher Salze, setzt man in der Regel in einer Konzentration von 1 Gew.-% bis zur Sättigungskonzentration des oder der entsprechenden Salze, bevorzugt von 5 bis 35, besonders bevorzugt von 15 bis 30 Gew.-%, ein.
   Die Stromdichten liegen im allgemeinen im Bereich von 0,5 bis 10, bevorzugt von 1 bis 4 kA/m². Die Zellspannung beträgt in der Regel pro Membranelektrolyseeinheit 2 bis 10 V, bevorzugt 3 bis 5 V.
   Die pH-Werte liegen im allgemeinen in den Anolytkammern im Bereich von 2 bis 10, bevorzugt 3 bis 5, in den Katolytkammern im Bereich größer als 13.
   Die Kammerbreite beträgt in der Regel 0,5 bis 10, bevorzugt 1 bis 5 mm.
   Die Temperatur während der Membranelektrolyse wählt im allgemeinen man im Bereich von 50 bis 110, vorzugsweise von 65 bis 90°C.
   Um den Stofftransport zu gewährleisten, wälzt man im allgemeinen die Kammerinhalte entweder mittels Pumpen oder im Naturumlauf, d.h. durch Mammutpumpeneffekt der Gasentwicklung an den Elektroden, um. Die Strömungsgeschwindigkeiten in den Kammern liegen im allgemeinen im Bereich von 0,05 bis 0,5, bevorzugt von 0,1 bis 0,2 m/sec.
e) Eine besonders bevorzugte Ausführungsform ist die elektrochemische Spaltung der Dicarbonsäuresalze in Dicarbonsäure und die betreffende Base in einer dreigeteilten Membranelektrolysezelle.
   Die dreigeteilte Membranelektrolyseeinheit ist in der Regel wie folgt aufgebaut.
   -Anode-Anolytkammer-Kationenaustauschermembran-Mittelkammer-Kationenaustauschermembran-Katolytkammer-Kathode.
   Die wäßrige Dicarbonsäuresalzlösung wird im allgemeinen der Mittelkammer zugeführt. Um die elektrische Leitfähigkeit in der Mittelkammer zu erhöhen, kann man eine Mineralsäure oder ein Salz dem Mittelkammerelektrolyten beigegeben. Beispielhaft genannt seien Schwefelsäure, Salpetersäure, Natriumsulfat und Natriumnitrat.
   In der Mittelkammer verbleibt im allgemeinen der organische Säurerest, der mit den Wasserstoffionen, die bei der Anodenreaktion frei werden und über die anodenseitige Kationenaustauschermembran in die Mittelkammer überführt werden, zu freier Säure reagieren kann. Die Säure wird in der Regel zusammen mit dem nicht umgesetzten Salz dem Mittelkammersystem entnommen. Als Anolyt kann man eine wäßrige Mineralsäure wie Schwefelsäure, Salpetersäure oder Salzsäure einsetzen, bevorzugt Schwefelsäure. Der Anolyt dient im wesentlichen zusammen mit der anodenseitigen Kationenaustauschermembran zum Schutz der organischen Dicarbonsäure vor anodischer Oxidation.
   Im übrigen kann man das Verfahren nach (e) unter den bei (d) genannten Bedingungen durchführen.
(f) Die elektrochemische Spaltung der Dicarbonsäuresalze in Dicarbonsäure und die betreffende Base kann man auch in einer viergeteilten Membranelektrolysezelle durchführen.
   Die viergeteilte Membranelektrolyseeinheit ist im allgemeinen wie folgt aufgebaut:
   -Anode-Anolytkammer-Kationenaustauschermembran-anodennahe Mittelkammer-Anionenaustauschermembran-kathodennahe Mittelkammer-Kationenaustauschermembran-Katolytkammer-Kathode.
   Die wäßrige Dicarbonsäuresalzlösung führt man zweckmäßig der Kathodennahen Mittelkammer zu.
   Um die elektrische Leitfähigkeit in der Mittelkammer zu erhöhen, kann man eine Mineralsäure oder ein Salz wie Schwefelsäure, Salpetersäure, Natriumsulfat oder Natriumnitrat dem Mittelkammerelektrolyten beigeben.
   Aus der kathodennahen Mittelkammer wird im allgemeinen das Säureanion in die anodennahe Mittelkammer überführt, wo es mit Wasserstoffionen, die bei der Anodenreaktion frei werden und über die anodenseitige Kationenaustauschermembran in die anodennahe Mittelkammer gelangen, zu freier Säure reagiert. Die Säure wird in der Regel mit hoher Reinheit dem Mittelkammersystem entnommen. Die Restsalzlösung wird im allgemeinen der kathodennahen Mittelkammer entnommen und im Teilstrom in die Adipatlösestufe zurückgeführt oder entsorgt. Als Anolyten setzt man in der Regel eine wäßrige Mineralsäure ein, bevorzugt Schwefelsäure. Der Anolyt dient im wesentlichen zusammen mit der anodenseitigen Kationenaustauschermembran zum Schutz der organischen Säure vor anodischer Oxidation.
   Im übrigen kann man das Verfahren nach (f) bei den bis (d) genannten Bedingungen durchführen.

Bei den vorgenannt beschriebenen Verfahrensvarianten verwendet man als Kationenaustauschermembranen besonders bevorzugt Polymere auf Basis von perfluorierten Olefinen oder Copolymere aus Styrol und Divinylbenzol, die als ladungstragende Gruppen Sulfonsäure- und gewünschtenfalls Carboxylgruppen enthalten. Ganz besonders bevorzugt verwendet man Membranen, die nur Sulfonsäuregruppen enthalten, da sie in der Regel beständiger gegenüber Verschmutzungen durch mehrwertige Kationen sind als andere Membranen. Solche Membranen sind bekannt (beispielsweise sogenannte Nafionmembranen vom Typ 324). Sie bestehen aus einem Copolymer von Tetrafluorethylen und einem perfluorierten Monomer, das Sulfongruppen enthält. Sie weisen in der Regel eine hohe chemische und thermische Stabilität auf. Zur mechanischen Verstärkung kann die Ionenaustauschermembran durch ein Teflonstützgewebe stabilisiert sein. Weiterhin sind Copolymere auf der Basis von Styrol und Divinylbenzol geeignet.

Als Anionenaustauschermembranen kann man beispielsweise die in der EP-A 449 071 ausführlich beschriebenen Membranen verwenden, so daß sich weitere Ausführungen hierzu erübrigen.

Als Elektrodenmaterialien kann man im allgemeinen perforierte Materialien verwenden, die z.B. in Form von Netzen, Lamellen, Ovalprofilstegen oder Rundprofilstegen ausgeführt sind.

Die Sauerstoffüberspannung der Anoden wählt man in der Regel im erfindungsgemäßen Stromdichtebereich weniger als 400 mV, damit es nicht zur Bildung von Ozon oder Perverbindungen kommt.

Geeignete Anodenmaterialien mit geringen Sauerstoffüberspannungen sind beispielsweise Titanträger mit elektrisch leitenden Zwischenschichten aus Boriden und/oder Carbiden und/oder Siliciden der IV. bis VI. Nebengruppe wie Tantalboride, Titanboride oder Titansuboxid sowie gegebenenfalls dotierte Zinnoxide oder gewünschtenfalls mit Platinmetallen dotiertes Tantal und/oder Niob, deren Oberfläche im allgemeinen mit elektrisch leitenden, nicht stöchiometrischen Mischoxiden der IV. bis VI. Nebengruppe und Metallen oder Metalloxiden der Platingruppe oder Platinmetallverbindungen wie Platinaten dotiert ist. Auf diesen Zwischenschichten befindet sich im allgemeinen das aktive Elektrodenmaterial, das bevorzugt aus Mischoxiden des Tantals mit Iridium, Platin oder Rhodium sowie Platinaten vom Typ Li_{0,3}Pt₃O₄ besteht. Zur Vergrößerung der Oberfläche verwendet man in der Regel oberflächlich aufgerauhte oder makroporöse Titanträger.

Als Kathoden setzt man im allgemeinen Elektrodenmaterialien mit einer niedrigen Wasserstoffüberspannung ein, um zusätzliche Spannungsverluste in der Membranelektrolyse- oder Elektrodialysezelle zu vermeiden. Geeignete Kathoden sind beispielsweise Eisen- oder Nickelträger, die oberflächlich mit feinteiligem Kobalt, Nickel, Molybdän, Wolfram, Mangan, Raneymetallverbindungen des Nickels oder Kobalts, Nickel- oder Kobalt-Aluminiumlegierungen, oder Nickel-Eisenlegierungen oder Kobalt-Eisenlegierungen mit 65 bis 90 Gew.-% Eisen beschichtet sind.

Zur Verbesserung der Selektivität und der Membranstandzeiten können auf der Kathodenseite Kationenaustauschermembranen mit Hydroxylionenblockern verwendet werden. Man kann die Selektivität ferner dadurch verbessern, daß man den Gehalt an Calcium-, Magnesium- und Aluminiumionen sowie den Kieselsäureanteil kleiner als jeweils 5 ppm hält.

Die nach der elektrochemischen Behandlung gewonnene Dicarbonsäure liegt im allgemeinen als wäßrige Lösung in einer Konzentration im Bereich von 1 bis 30, bevorzugt von 4 bis 30 Gew.-%, vor. Diese Lösung kann gegebenenfalls vorhandenes Leitfähigkeitssalz in einer Konzentration im Bereich von 0,05 bis 15, bevorzugt von 0,06 bis 6 Gew.-%, und gegebenenfalls vorhandene Mineralsäure in einer Konzentration im Bereich von 0,05 bis 15, bevorzugt von 0 bis 6 Gew.-%, enthalten.

Die erfindungsgemäß erhaltene Lauge enthält in der Regel ein Alkalimetallhydroxid in einer Konzentration im Bereich von 5 bis 35, vorzugsweise von 10 bis 25 Gew.-%.

Besonders bevorzugt kann man die erfindungsgemäß erhaltene Alkalimetallhydroxidlösung in den Kreislauf zurückführen oder anderweitig verwenden, wobei man sie gewünschtenfalls zuvor in an sich bekannter Weise, beispielsweise durch Eindampfen, aufkonzentrieren kann.

Um die Dicarbonsäure in reiner Form zu erhalten, kristallisiert man sie hierzu in der Regel aus der erfindungsgemäß erhaltenen Lösung aus, trennt das Produkt anschließend, beispielsweise durch Filtration, ab und trocknet es.

Vorzugsweise erhält man die Dicarbonsäure aus den bei der Elektrodialyse bzw. Membranelektrolyse erhaltenen Lösungen, durch Kühlungs- oder Verdampfungskristallisation. Anschließend trennt man im allgemeinen die Dicarbonsäuren aus den so erhaltenen Suspensionen ab, z.B. durch Filtration, Dekantieren oder Zentrifugieren.

Die Kühlungskristallisation führt man üblicherweise bei einer Temperatur im Bereich von 0 bis 50°C, bevorzugt von 10 bis 40°C, wobei man zweckmäßig bei Drücken im Bereich von 1 bis 100 kPa, vorzugsweise von 4 bis 20 kPa arbeitet, durch.

Die abgetrennten Dicarbonsäuren kann man vorzugsweise durch Waschen, beispielsweise mit Wasser oder C₁-C₄-Alkanolen, und gewünschtenfalls durch Umkristallisieren in reiner Form erhalten. Bei gleichzeitigem Vorliegen von mehreren Dicarbonsäuren kann man aufgrund der unterschiedlichen Löslichkeiten nach bekannten Methoden wie fraktioniertes Kristallisieren die einzelnen Dicarbonsäuren in reiner Form isolieren.

Die bei der Kristallisation und dem Waschen anfallenden wäßrigen Lösungen kann man wie üblich aufkonzentrieren und erneut der Kristallisation unterwerfen, beispielsweise dadurch, daß man sie zu noch zu kristallisierenden Lösungen, die direkt nach der Elektrodialyse bzw. Membranelektrolyse anfallen, hinzugibt. Man kann sie beispielsweise auch der festen Phase, die man nach der Behandlung der eingesetzten Polymerisate mit einer Base erhält, oder davon erhaltenen Gemischen, zuführen.

Ein Vorteil des erfindungsgemäßen Verfahrens gegenüber bekannten Verfahren liegt darin, daß die Bildung und Entsorgung von Salzen, die üblicherweise bei der Freisetzung der Dicarbonsäuren aus ihren Salzen durch Ansäuern anfallen, entfällt. Ein weiterer Vorteil besteht darin, daß auch faserverstärkte, mineralgefüllte und/oder schlagzähmodifizierte Formmassen aufgearbeitet werden können. Des weiteren kann man die durch das erfindungsgemäße Verfahren erhaltenen Stoffe wie Dicarbonsäuren, Diamine und Basen sowie gegebenenfalls Glasfasern und mineralische Füllstoffe zur Herstellung neuer Produkte verwenden.

### Vergleichsbeispiel

300 g eines auf ca. 8 mm (mittlerer Teilchendurchmesser) zerkleinertes Polyamid 66 mit einer Viskositätszahl (VZ) = 149 (Einheit: 1 cm³/g) (gemessen an einer 0,5 gew.-%igen Lösung des Polyamids in 96 gew.-%iger Schwefelsäure bei 25°C nach DIN 53 727) wurden in einem Druckbehälter unter Rühren mit 780 g einer 15 gew.-%igen Lösung von Natriumhydroxid in Methanol für 4 Stunden auf 180°C erhitzt.

Nach dem Abkühlen der Reaktionsmischung wurde das ausgefallene Natriumadipat abfiltriert, mehrmals mit Methanol gewaschen und getrocknet.

Das Mutterfiltrat und die vereinigten methanolischen Waschfiltrate wurden einer fraktionierten Destillation unterworfen. Zunächst wurden bei Normaldruck Leichtsieder wie Methanol abgetrennt. Bei 128-131°C/100 mbar wurden dann 142 g Hexamethylendiamin als farblose Schmelze erhalten.

249 g des getrockneten Natriumadipats wurden dann mit 673 g Wasser versetzt, so daß eine 27 gew.-%ige wäßrige Na-Adipat-Lösung erhalten wurde.

Diese konzentrierte Natriumadipatlösung wurde anschließend mit 0,5 g gepulverter Aktivkohle pro 100 ml Lösung versetzt und auf 50°C erwärmt. Nach 1 h wurde die Aktivkohle abfiltriert und unter Rühren wurden 80 mg Natriumcarbonat pro 100 g Lösung zugesetzt. Nach 1 h wurde der Rührer abgeschaltet und nach weiteren 4 h wurde die Lösung filtriert. Diese vorgereinigte Natriumadipatlösung wurde dann einer Behandlung mit einem Selektiv-Ionenaustauschharz (Lewatit TP 208 (von Bayer)) unterworfen.

### Beispiel 1

In einem Druckbehälter wurden unter Rühren 300 g zerkleinertes Polyamid 66 (wie in Vergleichsbeispiel beschrieben) mit 970 g einer 12,2 gew.-%igen Lösung von Natriumhydroxid in einer Lösemittelmischung, bestehend aus 85 Vol.-% Methanol und 15 Vol.-% Wasser, für 4 Stunden auf 180°C erhitzt.

Nach dem Abkühlen der Reaktionsmischung wurde das ausgefallene Na-Adipat abfiltriert, mehrmals mit insgesamt 750 g Methanol gewaschen und getrocknet. Die vereinigten methanolischen Waschfiltrate wurden als Lösemittel für die Hydrolysestufe wiederverwendet.

Das Mutterfiltrat der Reaktionsmischung wurde einer fraktionierten Destillation unterworfen. Dabei wurden zunächst unter Normaldruck Leichtsieder wie Methanol und Wasser abgetrennt. Bei 128-131°C/100 mbar wurden 138 g Hexamethylendiamin als farblose Schmelze erhalten.

Die Aufarbeitung des Na-Adipats zu Adipinsäure und Natronlauge erfolgte analog zum Vergleichsbeispiel, wobei die bei der Elektrolyse angefallene Natronlauge auf 50 Gew.-% aufkonzentriert und wieder in der Spaltreaktion (Hydrolysestufe) eingesetzt wurde. Dabei wurden erneut 300 g zerkleinertes Polyamid 66 (wie im Vergleichsbeispiel beschrieben) mit 232 g 50 gew.-%iger Natronlauge und 730 g des methanolischen Waschfiltrats unter Rühren für 4 Stunden auf 180°C erhitzt.

Nach Abkühlen der Reaktionsmischung wurde das ausgefallene Natriumadipat abfiltriert und mehrmals mit insgesamt 750 g Methanol gewaschen. Für dieses Waschen des Filterkuchens wurde Methanol verwendet, das aus der Rektifikation des Mutterfiltrats rein gewonnen wurde.
(Die weitere Aufarbeitung erfolgte analog zum Vergleichsbeispiel).

### Beispiel 2

Für diesen Versuch wurde ein auf ca. 8 mm (mittlerer Teilchendurchmesser) zerkleinertes, (mit Ruß) schwarz eingefärbtes, (wärmestabilisiertes) und glasfaserverstärktes Polyamid 66 mit einer Viskositätszahl (VZ) = 140 (gemessen nach DIN 53 727, s. Vergleichsbeispiel) und einem Glasfaseranteil von 36 Gew.-% (Bestimmung des Glühverlustes von glasfaserverstärkten Kunststoffen nach DIN 53 395) verwendet. In einem Druckbehälter wurden unter Rühren 490 g dieses Verbundmaterials mit 1180 g einer 10 gew.-%igen Lösung von Natriumhydroxid einer Lösemittelmischung bestehend aus 75 Vol.-% Methanol und 25 Vol.-% Wasser für 4 Stunden auf 180°C erhitzt.

Nach dem Abkühlen der Reaktionsmischung wurde das ausgefallene Natriumadipat zusammen mit den Glasfasern (und anderen unlöslichen Bestandteilen wie beispielsweise Rußpigmenten) abfiltriert und mehrmals mit Methanol gewaschen. Das Mutterfiltrat und die vereinigten Waschfiltrate wurden einer fraktionierten Destillation unterworfen. Zunächst wurden bei Normaldruck Leichtsieder wie Methanol und Wasser abgetrennt. Bei 128-131°C/100 mbar wurden 140 g Hexamethylendiamin als farblose Schmelze erhalten.

Zur Gewinnung des Natrium-Adipats wurde der Filterrückstand der Reaktionsmischung mehrmals mit insgesamt 1000 g Wasser versetzt, aufgerührt und filtriert. Aus den vereinigten Filtraten wurde eine 20 gew.-%ige wäßrige Na-Adipatlösung erhalten, die unter Normaldruck bis zu einer Konzentration von 27 Gew.-% Natriumadipat eingedampft wurde, wobei Reste an Methanol mitentfernt wurden.

Die so konzentrierte Na-Adipatlösung wurde anschließend mit 0,5 g gepulverter Aktivkohle pro 100 ml Lösung versetzt und auf 50°C erwärmt. Nach 1 h wurde die Aktivkohle abfiltriert.
(Die weitere Aufarbeitung erfolgte analog zum Vergleichsbeispiel.

### Beispiel 3

### Diskontinuierliche Eletrolyse in einer Dreikammerelektrolysezelle gemäß Variante e)

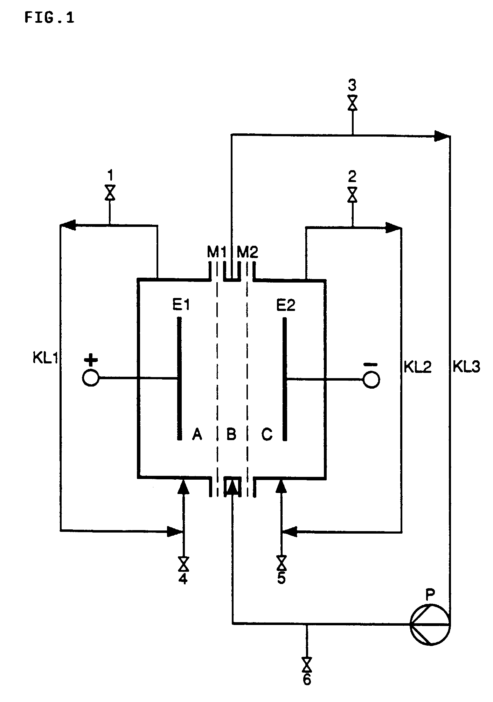

Verwendet wurde die in Figur 1 schematisch gezeigte Dreikammerelektrolysezelle mit drei Flüssigkeitskreisläufen (KL1 bis KL3). Alle produktberührenden Teile, mit Ausnahme der Elektroden, bestanden aus Polypropylen, Glas oder Quarz. Als Anode (E1) (in Kammer (A)) wurde eine Titanstreckmetallanode mit einer Fläche von 100 cm² mit einem für Sauerstoffentwicklung geeigneten Coating eingesetzt. Die Kathode (E2) (in Kammer (C)) hatte ebenfalls eine Fläche von 100 cm². Sie bestand aus einem Chrom-Nickel-Edelstahl (1,4571), der mit einem für die Wasserstoffentwicklung aktivierten Nickelnetz überzogen war. Die beiden Membranen (M1 und M2) vom Typ Nafion® 324 lagen direkt auf den Elektroden (E1 bzw. E2) auf und waren durch eine 1 mm breite Mittelkammer (B) mit einem Polypropylen-Spacer voneinander getrennt.

Die Anoden- (KL1) und Kathoden- (KL2) -Kreisläufe wurden aufgrund der Gasentwicklungen an den Elektroden in Naturumlauf gehalten. Der Kreislauf der Mittelkammer (B), (KL3), wurde mit einer Kreiselpumpe (P) umgewälzt. Die Strömungsgeschwindigkeit in der Mittelkammer (B) betrug 0,1 m/sec.

Eingesetzt wurden als Anolyt an der Stelle (1) 1131 g einer 5 gew.-%igen Schwefelsäure, als Katolyt an der Stelle (2) 1161 g einer 5 gew.-%igen Natronlauge und als Mittelkammerelektrolyt an der Stelle (3) 995 g der aus dem Vergleichsbeispiel erhaltenen 27 gew.-%igen Natriumadipatlosung, der 21 g einer 96 gew.-%igen Schwefelsäure zugesetzt wurden, so daß 1015 g einer Lösung, enthaltend 22 Gew.-% Natriumadipat, 2,9 Gew.-% Adipinsäure und 2,8 Gew.-% Natriumsulfat, eingesetzt wurden.

Bei einer Temperatur von 80°C, Atmosphärendruck, einer Stromdichte von 3,0 kA/m², einer Zellspannung von 4,0 V (zu Beginn) und 5,3 V (am Ende des Versuches) wurden bei einer Stromausbeute von 83 % nach einer Reaktionszeit von 2 h 26 min folgende Elektrolyte erhalten:
Anolyt (entnommen an Stelle (4)): 729 g einer 6,9 gew.-%igen Schwefelsäure,
Katolyt (entnommen an Stelle (5)): 1294 g einer 10,9 gew.-%igen Natronlauge,
Mittelkammelektrolyt (entnommen an Stelle (6)): 904 g einer Lösung, enthaltend 20,4 Gew.-% Adipinsäure, 1,2 Gew.-% Natriumadipat und 3,2 Gew.-% Natriumsulfat.

### Diskontinuierliche Kristallisation

900 g der so erhaltenen Mittelkammerelektrolytlösung wurden bei einer Temperatur von 80°C in ein Vakuumgefäß mit Rückflußkühler eingebracht und dann innerhalb von 100 min auf 10°C (durch stetiges Vermindern des Innendruckes (absolut) von 1013 mbar auf 12 mbar) abgekühlt. Die auskristallisierte Adipinsäure wurde anschließend mittels einer Vakuumnutsche bei einem Filtrationsdruck von 450 mbar abgetrennt und mit 700 g Wasser, das eine Temperatur nahe 0°C hatte, gewaschen. Das so gewaschene Kristallisat wurde nachfolgend in 420 g Wasser gelöst unter Erhalt einer 30 gew.-%igen Adipinsäurelösung. Danach wurde der Kristallisationsvorgang wiederholt. Nach dem Trocknen des nach dem zweiten Kristallisationsvorgang erhaltenen Kristallisats bei 80°C und 100 mbar Druck (absolut) wurden 175 g Adipinsäure mit einer Reinheit von 99,8 % und einem Aschegehalt von weniger als 8 ppm erhalten.

### Kontinuierliche Kristallisation

Beispiel 3 wurde wiederholt, die Reinigung der Adipinsäure erfolgte jedoch durch kontinuierliche Kristallisation. Hierzu wurden zwei Vakuumgefäße (0,75 l Nenninhalt, mit Rührvorrichtung) hintereinandergeschaltet. Der Absolutdruck der ersten Stufe (1. Gefäß) betrug 95 mbar (entsprechend einer Siedetemperatur der eingesetzten Adipatlösung von 45°C), der Absolutdruck der zweiten Stufe betrug 12 mbar (entsprechend einer Siedetemperatur der eingesetzten Adipatlösung von 10°C). Der Flüssigkeitsstand wurde in beiden Gefäßen konstant gehalten, indem mittels einer Membrandosierpumpe 0,75 kg/h Adipatlösung kontinuierlich in das erste Vakuumgefäß gepumpt und unter Flüssigkeitüberdeckung entspannt wurden. Mit Hilfe eines Standregelventils wurden ebenfalls 0,75 kg/h der im ersten Vakuumgefäß enthaltenen Lösung in das zweite Vakuumgefäß geleitet, wobei die vom ersten in das zweite Vakuumgefäß transportierte Lösung ebenfalls "getaucht" entspannt wurde. Eine Charge (900 g) der aus dem zweiten Gefäß auskristallisierten Adipinsäure wurde mittels einer Vakuumnutsche bei einem Filtrationsdruck von 450 mbar abgetrennt und mit 700 g Wasser, das eine Temperatur nahe 0°C hatte, gewaschen. Das so gewaschene Kristallisat wurde nachfolgend in 420 g Wasser gelöst unter Erhalt einer 30 gew.-%igen Adipinsäurelösung. Danach wurde der Kristallisationsvorgang wiederholt. Nach dem Trocknen des nach dem zweiten Krstallisationsvorgang erhaltenen Kristallisats bei 80°C und 100 mbar Druck (absolut) wurden 175 g Adipinsäure mit einer Reinheit von 99,8 % und einem Aschegehalt von weniger als 8 ppm erhalten.

### Beispiel 4

### Diskontinuierliche Elektrolyse in einer Vierkammerelektrolysezelle gemäß Variante f)

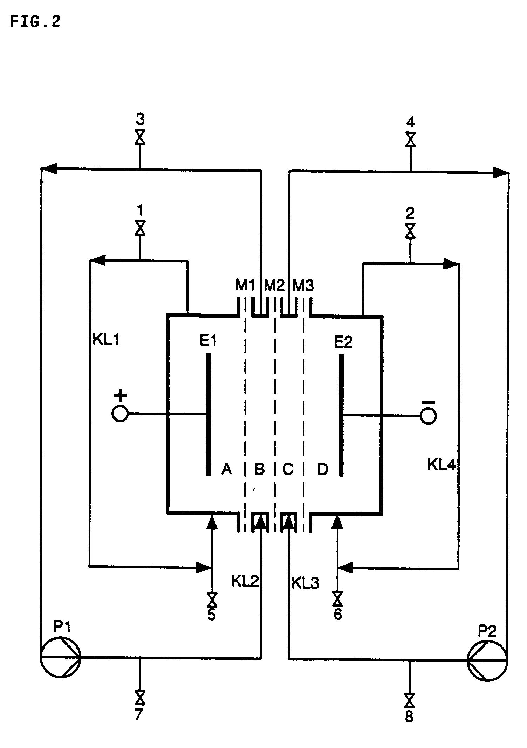

Verwendet wurde die in Figur 2 schematisch gezeigte Vierkammerelektrolysezelle mit vier Flüssigkeitskreisläufen (KL1 bis KL4). Alle produktberührenden Teile, mit Ausnahme der Elektroden, bestanden aus Polypropylen, Glas oder Quarz. Als Anode (E1) (in Kammer (A)) wurde eine Titanstreckmetallanode mit einer Fläche von 100 cm² mit einem für Sauerstoffentwicklung geeigneten Coating eingesetzt. Die Kathode (E2) (in Kammer (D)) hatte ebenfalls eine Fläche von 100 cm². Sie bestand aus einem Chrom-Nickel-Edelstahl (1,4571)), der mit einem für die Wasserstoffentwicklung aktivierten Nickelnetz überzogen war. Die beiden elektrodennahen Kationenaustauscher-Membranen (M1 und M3) vom Typ Nafion® 324 lagen direkt auf den Elektroden (E1 bzw. E2) auf und waren durch zwei jeweils 1 mm breite Mittelkammern, (B) und (C), mit einer in der Mitte angeordneten Anionenaustauschermembran (M2) vom Typ Tokuyama Soda® AMH getrennt. Die Mittelkammern, (B) und (C), wurden mit zwei Polypropylen-Spacern versehen, die dazu dienten, den Strömungskanal freizuhalten und den direkten Kontakt der Membranen zu verhindern.

Die Anoden- (KL1) und Kathoden- (KL4) -Kreisläufe wurden aufgrund der Gasentwicklungen an den Elektroden in Naturumlauf gehalten. Die Kreisläufe der Mittelkammern (B) und (C), (KL2) und (KL3), wurden mit den Kreiselpumpen (P1) und (P2) umgewälzt. Die Strömungsgeschwindigkeiten in den Mittelkammern (B) und (C) betrugen jeweils 0,1 m/sec.

Eingesetzt wurden als Anolyt an der Stelle (1) 1108 g einer 5,1 gew.-%igen Schwefelsäure,
als Katolyt an der Stelle (2) 1101 g einer 4 gew.-%igen Natronlauge, und als Elektrolyt der anodennahen Mittelkammer (B) an der Stelle (3) 1097 g einer 2,1 gew.-%igen Schwefelsäure,
als Elektrolyt der Kathodennahen Mittelkammer (C) an der Stelle (4) 1505 g der aus Beispiel 2 erhaltenen 27 gew.-%igen Natriumadipatlösung.

Während der Reaktion wurden insgesamt noch 900 g Wasser in die Kathodennahe Mittelkammer (C) gegeben.

Bei einer Temperatur von 80°C, Atmosphärendruck, einer Stromdichte von 3,0 kA/m², einer Zellspannung von 7,0 V (zu Beginn) und 8,7 V (am Ende des Versuches) wurden bei einer Stromausbeute von 75 % nach einer Reaktionszeit von 5 h, wobei der pH-Wert in der kathodennahen Mittelkammer (C) im Bereich von 10 bis 12 lag, folgende Elektrolyte erhalten:
Anolyt (entnommen an Stelle (5)): 793 g einer 7,1 gew.-%igen Schwefelsäure,
Katolyt (entnommen an Stelle (6)): 1584 g einer 13,3 gew.-%igen Natronlauge,
Produkt der anodennahen Mittelkammer (B) (entnommen an Stelle (7)): 2034 g einer Lösung, enthaltend 15,1 Gew.-% Adipinsäure, 1,1 Gew.-% Schwefelsäure,
Produkt der kathodennahen Mittelkammer (C) (entnommen an Stelle (8)): 1061 g einer 1,4 gew.-%igen Natriumadipatlösung.

### Beispiel 5

### Diskontinuierliche Elektrolyse in einer Membranstapelzelle gemäß Variante b)

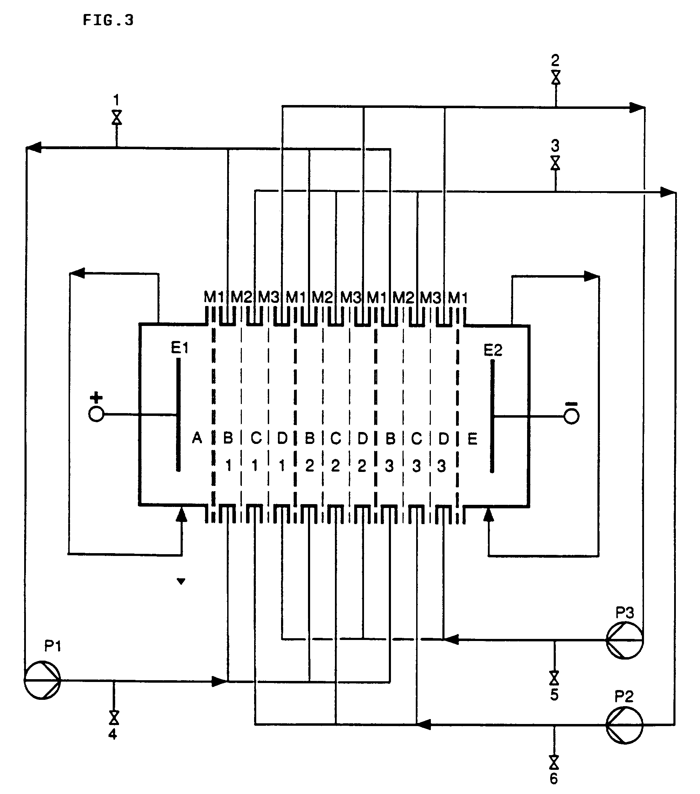

Verwendet wurde die in Figur 3 schematisch gezeigte Membranstapelzelle mit drei Flüssigkeitskreisläufen (KL1 bis KL3). Alle produktberührenden Teile, mit Ausnahme der Elektroden, bestanden aus Polypropylen, Glas oder Polytetrafluorethylen. Als Anode (E1) (in Kammer (A)) wurde eine Titanstreckmetallanode mit einer Fläche von 320 cm² mit einem für Sauerstoffentwicklung geeigneten Coating eingesetzt. Die Kathode (E2) (in Kammer (D)) hatte ebenfalls eine Fläche von 320 cm². Sie bestand aus einem Chrom-Nickel-Edelstahl (1,4571), der mit einem für die Wasserstoffentwicklung aktivierten Nickelnetz überzogen war.

Die Kammern (A) und (B1), (D1) und (B2), (D2) und (B3) sowie (D3) und E wurden jeweils durch eine bipolare Membran (aus DE-A 40 26 154) voneinander getrennt. Die Kammern (B1) und C1), (B2) und (C2) sowie (B3) und (C3) waren durch Anionenaustauschermembranen (Tokuyama® Soda AMX) voneinander getrennt. Die Kammern (C1) und (D1), (C2) und (D2) sowie (C3) und (D3) waren durch Kationenaustauschermembranen (Tokuyama® Soda CMX) voneinander getrennt. Die Membranabstände betrugen jeweils 0,5 mm.

Alle Flüssigkeitskreisläufe, mit Ausnahme derjenigen der Anoden- (A) und Kathoden- (E) -Kammern, wurden mittels Kreiselpumpen, (P1) bis (P3), umgewälzt, wobei die Strömungsgeschwindigkeiten jeweils 0,1 m/sec betrugen.

Eingesetzt wurden als Elektrolyt im sauren Milieu an der Stelle (1) 10 000 g einer 1,5 gew.-%igen Schwefelsäure,
als Elektrolyt im basischen Milieu an der Stelle (2) 5000 g einer 1 gew.-%igen Natronlauge, und
als Mittelkammerelektrolyt an Stelle (3) 5000 g einer aus Beispiel 2 erhaltenen 20 gew.-%igen Natriumadipatlösung (erhalten durch Verdünnen der in Beispiel 2 erhaltenen 27 gew.-%igen Lösung).

Bei einer Temperatur von 55°C, Atmosphärendruck, einer Stromdichte von 0,31 kA/m², einer Zellspannung von 13 V (im Mittel) wurden bei einer Stromausbeute von 70 % nach einer Reaktionszeit von 13 h folgende Elektrolyte erhalten.

Dialyseprodukt (entnommen an Stelle (4)): 11 555 g einer 6,4 gew.-%igen Adipinsäure, die zusätzlich 1,3 Gew.-% Schwefelsäure enthielt,
"basischer" Elektrolyt (entnommen an Stelle (5)): 6597 g einer 6,9 gew.-%igen Natronlauge,
"abgereicherter" Mittelkammerelektrolyt (entnommen an Stelle (6)): eine 1,8 gew.-%ige Natriumadipatlösung.

## Patentansprüche

1. Verfahren zur gleichzeitigen Herstellung von Dicarbonsäuren und Diaminen aus
(a) Polymerisaten auf der Basis von Polyamiden aus Dicarbonsäuren oder deren Derivaten mit Diaminen, oder
) (b) Massen, enthaltend im wesentlichen solche Polymerisate,
gekennzeichnet durch
(1) Spaltung dieser Polymerisate in ihre monomeren Bestandteile mit einer Base in einer Alkohol-Wasser Mischung, die 5 bis 40 Gew.-% Wasser enthält, unter Erhalt einer flüssigen und einer festen Phase,
(2) Abtrennung der festen von der flüssigen Phase unter Erhalt einer flüssigen Phase, die das Diamin enthält, und
(3) Elektrochemische Überführung der bei der Spaltung erhaltenen Dicarbonsäuresalze der Adipinsäure oder Sebacinsäure in die entsprechenden Dicarbonsäuren und Basen.

2. Verfahren gemäß Anspruch 1, dadurch gekennzeichnet, daß man die elektrochemische Überführung bei einer Temperatur im Bereich von 65 bis 90°C in einer drei- oder viergeteilten Membranelektrolysezelle vornimmt.

3. Verfahren gemäß Anspruch 1, dadurch gekennzeichnet, daß man die elektrochemische Überführung in einer Elektrodialyseeinheit, die aus drei Kammern besteht, bei einer Temperatur im Bereich von 40 bis 110°C vornimmt.

4. Verfahren gemäß den Ansprüchen 1 bis 3, dadurch gekennzeichnet, daß man die Adipinsäure oder Sebacinsäure aus ihren bei der elektrochemischen Behandlung erhaltenen Lösungen auskristallisiert.

5. Verfahren gemäß den Ansprüchen 1 bis 4, dadurch gekennzeichnet, daß man die bei der elektrochemischen Behandlung gewonnene Base zur Spaltung der Polymerisate in ihre monomeren Bestandteile einsetzt.

6. Verwendung der Verfahren gemäß den Ansprüchen 1 bis 5 zur Rückspaltung von Polyamiden in ihre monomeren Bestandteile.

## Claims

1. A process for the simultaneous production of dicarboxylic acids and diamines from
(a) polymers based on polyamides of dicarboxylic acids or their derivatives with diamines, or
(b) materials comprising essentially such polymers,
which comprises
(1) cracking these polymers into their monomeric constituents using a base in an alcohol-water mixture comprising from 5 to 40% by weight of water to obtain a liquid phase and a solid phase,
(2) separating the solid phase from the liquid phase to obtain a liquid phase comprising the diamine, and
(3) electrochemically converting the resulting dicarboxylic acid salts of adipic acid or sebacic acid into the corresponding dicarboxylic acids and bases.

2. A process as claimed in claim 1, wherein the electrochemical conversion is carried out in a three- or four-part membrane electrolysis cell at a temperature within the range from 65 to 90°C.

3. A process as claimed in claim 1, wherein the electrochemical conversion is carried out in an electrodialysis unit consisting of three compartments at a temperature within the range from 40 to 110°C.

4. A process as claimed in any of claims 1 to 3, wherein the adipic acid or sebacic acid is crystallized out from their solutions obtained by the electrochemical treatment.

5. A process as claimed in any of claims 1 to 4, wherein the base obtained in the electrochemical treatment is used for cracking the polymers into their monomeric constituents.

6. The use of a process as claimed in any of claims 1 to 5 for the regeneration cracking of polyamides into their monomeric constituents.

## Revendications

1. Procédé de préparation simultanée d'acides dicarboxyliques et de diamines à partir
(a) de polymères à base de polyamides formés à partir d'acides dicarboxyliques ou de leurs dérivés et de diamines, ou
(b) de masses, contenant principalement de tels polymères,
caractérisé par
(1) la dégradation de ces polymères en leurs constituants monomères, à l'aide d'une base dans un mélange alcool/eau contenant 5-40% en poids d'eau, pour donner une phase solide et une phase liquide,
(2) la séparation de la phase solide et de la phase liquide pour donner une phase liquide contenant la diamine et
(3) la conversion électrochimique des sels d'acides dicarboxyliques de l'acide adipique ou l'acide sébacique, obtenus lors de la dégradation, en les acides dicarboxyliques et bases correspondants.

2. Procédé selon la revendication 1, caractérisé en ce que l'on effectue la conversion électrochimique à des températures de 65-90°C dans une cellule d'électrolyse à membrane en trois ou quatre parties.

3. Procédé selon la revendication 1, caractérisé en ce que l'on effectue la conversion électrochimique à des températures de 40-110°C dans une unité d'électrodialyse constituée de trois chambres.

4. Procédé selon l'une quelconque des revendications 1 à 3, caractérisé en ce que l'on recristallise l'acide adipique ou l'acide sébacique à partir de leurs solutions obtenues lors du traitement électrochimique.

5. Procédé selon l'une quelconque des revendications 1 à 4, caractérisé en ce que l'on utilise la base obtenue lors du traitement électrochimique, pour la dégradation des polymères en leurs constituants monomères.

6. Utilisation du procédé selon l'une quelconque des revendications 1 à 5 pour la dégradation de polyamides en leurs constituants monomères.
